# EUROPEAN PATENT APPLICATION

(11) **EP 3 372 689 A1**
(43) Date of publication of application: **12.09.2018**
(21) Application number: 18169060.3
(22) Date of filing: 16.12.2010
(51) Int. Cl.: C12N 15/82, A01H 5/00, A01H 5/10, A01N 63/02, A01N 65/00

(54) **INSECTICIDAL PROTEIN COMBINATIONS FOR CONTROLLING FALL ARMYWORM AND EUROPEAN CORN BORER, AND METHODS FOR INSECT RESISTANCE MANAGEMENTS**

(30) Priority: 16.12.2009 US 28427809 P
(62) Divisional of application: 10838258.1
(71) Applicant: Dow AgroSciences LLC, Indianapolis IN 46268-1054 (US)
(72) Inventor: Meade, Thomas, Zionsville, IN 46077 (US); Narva, Kenneth E., Zionsville, IN 46077 (US); Storer, Nicholas P., Kensington, MD 20895 (US); Sheets, Joel J., Zionsville, IN 46077 (US); Burton, Stephanie L., Indianapolis, IN 46278 (US)
(74) Representative: Zwicker, Jörk

(57) **Abstract**

The subject invention relates in part to stacking certain Cry genes along with Cry 1Fa to result in products that are more durable and less prone towards insects developing resistance towards the activity of any of the toxins (such as Cry 1Fa) by itself. Embodiments of the Cry 1Fa stacking partners include Cry1Ea. These stacks can be used to control FAW as described herein.

SEQUENCE LISTING

<110>  Dow AGROSCIENCES LLC

<120>  COMBINED USE OF CRY1FA, CRY2AA, CRY1IA AND CRY1EA FOR MANAGEMENT OF
RESISTANT INSECTS

<130>  659-20 EPT1

<150>  10 838 258.1

<151>  16.12.2010

<160>  4

<170>  PatentIn version 3.5

<210>  1
<211>  605
<212>  PRT
<213>  Artificial Sequence

<220>
<223>  Cry1Fa

<400>  1

Met Glu Asn Asn Ile Gln Asn Gln Cys Val Pro Tyr Asn Cys Leu Asn
1               5                   10                  15

Asn Pro Glu Val Glu Ile Leu Asn Glu Glu Arg Ser Thr Gly Arg Leu
            20                  25                  30

Pro Leu Asp Ile Ser Leu Ser Leu Thr Arg Phe Leu Leu Ser Glu Phe
        35                  40                  45

Val Pro Gly Val Gly Val Ala Phe Gly Leu Phe Asp Leu Ile Trp Gly
    50                  55                  60

Phe Ile Thr Pro Ser Asp Trp Ser Leu Phe Leu Leu Gln Ile Glu Gln
65                  70                  75                  80

Leu Ile Glu Gln Arg Ile Glu Thr Leu Glu Arg Asn Arg Ala Ile Thr
                85                  90                  95

Thr Leu Arg Gly Leu Ala Asp Ser Tyr Glu Ile Tyr Ile Glu Ala Leu
            100                 105                 110

Arg Glu Trp Glu Ala Asn Pro Asn Asn Ala Gln Leu Arg Glu Asp Val
        115                 120                 125

Arg Ile Arg Phe Ala Asn Thr Asp Asp Ala Leu Ile Thr Ala Ile Asn
    130                 135                 140

Asn Phe Thr Leu Thr Ser Phe Glu Ile Pro Leu Leu Ser Val Tyr Val
145                 150                 155                 160

Gln Ala Ala Asn Leu His Leu Ser Leu Leu Arg Asp Ala Val Ser Phe
                165                 170                 175

Gly Gln Gly Trp Gly Leu Asp Ile Ala Thr Val Asn Asn His Tyr Asn
            180                 185                 190

Arg Leu Ile Asn Leu Ile His Arg Tyr Thr Lys His Cys Leu Asp Thr
        195                 200                 205

Tyr Asn Gln Gly Leu Glu Asn Leu Arg Gly Thr Asn Thr Arg Gln Trp
    210                 215                 220

Ala Arg Phe Asn Gln Phe Arg Arg Asp Leu Thr Leu Thr Val Leu Asp
225                 230                 235                 240

Ile Val Ala Leu Phe Pro Asn Tyr Asp Val Arg Thr Tyr Pro Ile Gln
                245                 250                 255

Thr Ser Ser Gln Leu Thr Arg Glu Ile Tyr Thr Ser Ser Val Ile Glu
            260                 265                 270

Asp Ser Pro Val Ser Ala Asn Ile Pro Asn Gly Phe Asn Arg Ala Glu
        275                 280                 285

Phe Gly Val Arg Pro Pro His Leu Met Asp Phe Met Asn Ser Leu Phe
    290                 295                 300

Val Thr Ala Glu Thr Val Arg Ser Gln Thr Val Trp Gly Gly His Leu
305                 310                 315                 320

Val Ser Ser Arg Asn Thr Ala Gly Asn Arg Ile Asn Phe Pro Ser Tyr
                325                 330                 335

Gly Val Phe Asn Pro Gly Gly Ala Ile Trp Ile Ala Asp Glu Asp Pro
            340                 345                 350

Arg Pro Phe Tyr Arg Thr Leu Ser Asp Pro Val Phe Val Arg Gly Gly
        355                 360                 365

Phe Gly Asn Pro His Tyr Val Leu Gly Leu Arg Gly Val Ala Phe Gln
    370                 375                 380

Gln Thr Gly Thr Asn His Thr Arg Thr Phe Arg Asn Ser Gly Thr Ile
385                 390                 395                 400

Asp Ser Leu Asp Glu Ile Pro Pro Gln Asp Asn Ser Gly Ala Pro Trp
                405                 410                 415

Asn Asp Tyr Ser His Val Leu Asn His Val Thr Phe Val Arg Trp Pro
            420                 425                 430

Gly Glu Ile Ser Gly Ser Asp Ser Trp Arg Ala Pro Met Phe Ser Trp
        435                 440                 445

Thr His Arg Ser Ala Thr Pro Thr Asn Thr Ile Asp Pro Glu Arg Ile
    450                 455                 460

Thr Gln Ile Pro Leu Val Lys Ala His Thr Leu Gln Ser Gly Thr Thr
465                 470                 475                 480

Val Val Arg Gly Pro Gly Phe Thr Gly Gly Asp Ile Leu Arg Arg Thr
                485                 490                 495

Ser Gly Gly Pro Phe Ala Tyr Thr Ile Val Asn Ile Asn Gly Gln Leu
            500                 505                 510

Pro Gln Arg Tyr Arg Ala Arg Ile Arg Tyr Ala Ser Thr Thr Asn Leu
        515                 520                 525

Arg Ile Tyr Val Thr Val Ala Gly Glu Arg Ile Phe Ala Gly Gln Phe
    530                 535                 540

Asn Lys Thr Met Asp Thr Gly Asp Pro Leu Thr Phe Gln Ser Phe Ser
545                 550                 555                 560

Tyr Ala Thr Ile Asn Thr Ala Phe Thr Phe Pro Met Ser Gln Ser Ser
                565                 570                 575

Phe Thr Val Gly Ala Asp Thr Phe Ser Ser Gly Asn Glu Val Tyr Ile
            580                 585                 590

Asp Arg Phe Glu Leu Ile Pro Val Thr Ala Thr Leu Glu
        595                 600                 605

<210>  2
<211>  633
<212>  PRT
<213>  Artificial Sequence

<220>
<223>  Cry2Aa

<400>  2

Met Asn Asn Val Leu Asn Ser Gly Arg Thr Thr Ile Cys Asp Ala Tyr
1               5                   10                  15

Asn Val Val Ala His Asp Pro Phe Ser Phe Glu His Lys Ser Leu Asp
            20                  25                  30

Thr Ile Gln Lys Glu Trp Met Glu Trp Lys Arg Thr Asp His Ser Leu
        35                  40                  45

Tyr Val Ala Pro Val Val Gly Thr Val Ser Ser Phe Leu Leu Lys Lys
    50                  55                  60

Val Gly Ser Leu Ile Gly Lys Arg Ile Leu Ser Glu Leu Trp Gly Ile
65                  70                  75                  80

Ile Phe Pro Ser Gly Ser Thr Asn Leu Met Gln Asp Ile Leu Arg Glu
                85                  90                  95

Thr Glu Gln Phe Leu Asn Gln Arg Leu Asn Thr Asp Thr Leu Ala Arg
            100                 105                 110

Val Asn Ala Glu Leu Ile Gly Leu Gln Ala Asn Ile Arg Glu Phe Asn
        115                 120                 125

Gln Gln Val Asp Asn Phe Leu Asn Pro Thr Gln Asn Pro Val Pro Leu
    130                 135                 140

Ser Ile Thr Ser Ser Val Asn Thr Met Gln Gln Leu Phe Leu Asn Arg
145                 150                 155                 160

Leu Pro Gln Phe Gln Ile Gln Gly Tyr Gln Leu Leu Leu Leu Pro Leu
                165                 170                 175

Phe Ala Gln Ala Ala Asn Met His Leu Ser Phe Ile Arg Asp Val Ile
            180                 185                 190

Leu Asn Ala Asp Glu Trp Gly Ile Ser Ala Ala Thr Leu Arg Thr Tyr
        195                 200                 205

Arg Asp Tyr Leu Arg Asn Tyr Thr Arg Asp Tyr Ser Asn Tyr Cys Ile
    210                 215                 220

Asn Thr Tyr Gln Thr Ala Phe Arg Gly Leu Asn Thr Arg Leu His Asp
225                 230                 235                 240

Met Leu Glu Phe Arg Thr Tyr Met Phe Leu Asn Val Phe Glu Tyr Val
                245                 250                 255

Ser Ile Trp Ser Leu Phe Lys Tyr Gln Ser Leu Met Val Ser Ser Gly
        260                 265                 270

Ala Asn Leu Tyr Ala Ser Gly Ser Gly Pro Gln Gln Thr Gln Ser Phe
        275                 280                 285

Thr Ala Gln Asn Trp Pro Phe Leu Tyr Ser Leu Phe Gln Val Asn Ser
    290                 295                 300

Asn Tyr Ile Leu Ser Gly Ile Ser Gly Thr Arg Leu Ser Ile Thr Phe
305                 310                 315                 320

Pro Asn Ile Gly Gly Leu Pro Gly Ser Thr Thr Thr His Ser Leu Asn
                325                 330                 335

Ser Ala Arg Val Asn Tyr Ser Gly Gly Val Ser Ser Gly Leu Ile Gly
            340                 345                 350

Ala Thr Asn Leu Asn His Asn Phe Asn Cys Ser Thr Val Leu Pro Pro
        355                 360                 365

Leu Ser Thr Pro Phe Val Arg Ser Trp Leu Asp Ser Gly Thr Asp Arg
    370                 375                 380

Glu Gly Val Ala Thr Ser Thr Asn Trp Gln Thr Glu Ser Phe Gln Thr
385                 390                 395                 400

Thr Leu Ser Leu Arg Cys Gly Ala Phe Ser Ala Arg Gly Asn Ser Asn
                405                 410                 415

Tyr Phe Pro Asp Tyr Phe Ile Arg Asn Ile Ser Gly Val Pro Leu Val
            420                 425                 430

Ile Arg Asn Glu Asp Leu Thr Arg Pro Leu His Tyr Asn Gln Ile Arg
        435                 440                 445

Asn Ile Glu Ser Pro Ser Gly Thr Pro Gly Gly Ala Arg Ala Tyr Leu
    450                 455                 460

Val Ser Val His Asn Arg Lys Asn Asn Ile Tyr Ala Ala Asn Glu Asn
465                 470                 475                 480

Gly Thr Met Ile His Leu Ala Pro Glu Asp Tyr Thr Gly Phe Thr Ile
                485                 490                 495

Ser Pro Ile His Ala Thr Gln Val Asn Asn Gln Thr Arg Thr Phe Ile
            500                 505                 510

Ser Glu Lys Phe Gly Asn Gln Gly Asp Ser Leu Arg Phe Glu Gln Ser
        515                 520                 525

Asn Thr Thr Ala Arg Tyr Thr Leu Arg Gly Asn Gly Asn Ser Tyr Asn
    530                 535                 540

Leu Tyr Leu Arg Val Ser Ser Ile Gly Asn Ser Thr Ile Arg Val Thr
545                 550                 555                 560

Ile Asn Gly Arg Val Tyr Thr Val Ser Asn Val Asn Thr Thr Thr Asn
                565                 570                 575

Asn Asp Gly Val Asn Asp Asn Gly Ala Arg Phe Ser Asp Ile Asn Ile
            580                 585                 590

Gly Asn Ile Val Ala Ser Asp Asn Thr Asn Val Thr Leu Asp Ile Asn
        595                 600                 605

Val Thr Leu Asn Ser Gly Thr Pro Phe Asp Leu Met Asn Ile Met Phe
    610                 615                 620

Val Pro Thr Asn Leu Pro Pro Leu Tyr
625                 630

<210>  3
<211>  648
<212>  PRT
<213>  Artificial Sequence

<220>
<223>  Cry1Ia

<400>  3

Met Lys Leu Lys Asn Gln Asp Lys His Gln Ser Phe Ser Ser Asn Ala
1               5                   10                  15

Lys Val Asp Lys Ile Ser Thr Asp Ser Leu Lys Asn Glu Thr Asp Ile
            20                  25                  30

Glu Leu Gln Asn Ile Asn His Glu Asp Cys Leu Lys Met Ser Glu Tyr
        35                  40                  45

Glu Asn Val Glu Pro Phe Val Ser Ala Ser Thr Ile Gln Thr Gly Ile
    50                  55                  60

Gly Ile Ala Gly Lys Ile Leu Gly Thr Leu Gly Val Pro Phe Ala Gly
65                  70                  75                  80

Gln Val Ala Ser Leu Tyr Ser Phe Ile Leu Gly Glu Leu Trp Pro Lys
                85                  90                  95

Gly Lys Asn Gln Trp Glu Ile Phe Met Glu His Val Glu Glu Ile Ile
            100                 105                 110

Asn Gln Lys Ile Ser Thr Tyr Ala Arg Asn Lys Ala Leu Thr Asp Leu
        115                 120                 125

Lys Gly Leu Gly Asp Ala Leu Ala Val Tyr His Asp Ser Leu Glu Ser
    130                 135                 140

Trp Val Gly Asn Arg Asn Asn Thr Arg Ala Arg Ser Val Val Lys Ser
145                 150                 155                 160

Gln Tyr Ile Ala Leu Glu Leu Met Phe Val Gln Lys Leu Pro Ser Phe
                165                 170                 175

Ala Val Ser Gly Glu Glu Val Pro Leu Leu Pro Ile Tyr Ala Gln Ala
            180                 185                 190

Ala Asn Leu His Leu Leu Leu Leu Arg Asp Ala Ser Ile Phe Gly Lys
        195                 200                 205

Glu Trp Gly Leu Ser Ser Ser Glu Ile Ser Thr Phe Tyr Asn Arg Gln
    210                 215                 220

Val Glu Arg Ala Gly Asp Tyr Ser Tyr His Cys Val Lys Trp Tyr Ser
225                 230                 235                 240

Thr Gly Leu Asn Asn Leu Arg Gly Thr Asn Ala Glu Ser Trp Val Arg
                245                 250                 255

Tyr Asn Gln Phe Arg Arg Asp Met Thr Leu Met Val Leu Asp Leu Val
            260                 265                 270

Ala Leu Phe Pro Ser Tyr Asp Thr Gln Met Tyr Pro Ile Lys Thr Thr
        275                 280                 285

Ala Gln Leu Thr Arg Glu Val Tyr Thr Asp Ala Ile Gly Thr Val His
    290                 295                 300

Pro His Pro Ser Phe Thr Ser Thr Thr Trp Tyr Asn Asn Asn Ala Pro
305                 310                 315                 320

Ser Phe Ser Ala Ile Glu Ala Ala Val Val Arg Asn Pro His Leu Leu
                325                 330                 335

Asp Phe Leu Glu Gln Val Thr Ile Tyr Ser Leu Leu Ser Arg Trp Ser
        340                 345                 350

Asn Thr Gln Tyr Met Asn Met Trp Gly Gly His Lys Leu Glu Phe Arg
        355                 360                 365

Thr Ile Gly Gly Thr Leu Asn Ile Ser Thr Gln Gly Ser Thr Asn Thr
    370                 375                 380

Ser Ile Asn Pro Val Thr Leu Pro Phe Thr Ser Arg Asp Val Tyr Arg
385                 390                 395                 400

Thr Glu Ser Leu Ala Gly Leu Asn Leu Phe Leu Thr Gln Pro Val Asn
                405                 410                 415

Gly Val Pro Arg Val Asp Phe His Trp Lys Phe Val Thr His Pro Ile
            420                 425                 430

Ala Ser Asp Asn Phe Tyr Tyr Pro Gly Tyr Ala Gly Ile Gly Thr Gln
        435                 440                 445

Leu Gln Asp Ser Glu Asn Glu Leu Pro Pro Glu Ala Thr Gly Gln Pro
    450                 455                 460

Asn Tyr Glu Ser Tyr Ser His Arg Leu Ser His Ile Gly Leu Ile Ser
465                 470                 475                 480

Ala Ser His Val Lys Ala Leu Val Tyr Ser Trp Thr His Arg Ser Ala
                485                 490                 495

Asp Arg Thr Asn Thr Ile Glu Pro Asn Ser Ile Thr Gln Ile Pro Leu
            500                 505                 510

Val Lys Ala Phe Asn Leu Ser Ser Gly Ala Ala Val Val Arg Gly Pro
        515                 520                 525

Gly Phe Thr Gly Gly Asp Ile Leu Arg Arg Thr Asn Thr Gly Thr Phe
    530                 535                 540

Gly Asp Ile Arg Val Asn Ile Asn Pro Pro Phe Ala Gln Arg Tyr Arg
545                 550                 555                 560

Val Arg Ile Arg Tyr Ala Ser Thr Thr Asp Leu Gln Phe His Thr Ser
                565                 570                 575

Ile Asn Gly Lys Ala Ile Asn Gln Gly Asn Phe Ser Ala Thr Met Asn

580                 585                 590

Arg Gly Glu Asp Leu Asp Tyr Lys Thr Phe Arg Thr Val Gly Phe Thr
        595                 600                 605

Thr Pro Phe Ser Phe Leu Asp Val Gln Ser Thr Phe Thr Ile Gly Ala
    610                 615                 620

Trp Asn Phe Ser Ser Gly Asn Glu Val Tyr Ile Asp Arg Ile Glu Phe
625                 630                 635                 640

Val Pro Val Glu Val Thr Tyr Glu
                645

<210>  4
<211>  603
<212>  PRT
<213>  Artificial Sequence

<220>
<223>  Cry1Ea

<400>  4

Met Glu Ile Val Asn Asn Gln Asn Gln Cys Val Pro Tyr Asn Cys Leu
1               5                   10                  15

Asn Asn Pro Glu Asn Glu Ile Leu Asp Ile Glu Arg Ser Asn Ser Thr
            20                  25                  30

Val Ala Thr Asn Ile Ala Leu Glu Ile Ser Arg Leu Leu Ala Ser Ala
        35                  40                  45

Thr Pro Ile Gly Gly Ile Leu Leu Gly Leu Phe Asp Ala Ile Trp Gly
    50                  55                  60

Ser Ile Gly Pro Ser Gln Trp Asp Leu Phe Leu Glu Gln Ile Glu Leu
65                  70                  75                  80

Leu Ile Asp Gln Lys Ile Glu Glu Phe Ala Arg Asn Gln Ala Ile Ser
                85                  90                  95

Arg Leu Glu Gly Ile Ser Ser Leu Tyr Gly Ile Tyr Thr Glu Ala Phe
            100                 105                 110

Arg Glu Trp Glu Ala Asp Pro Thr Asn Pro Ala Leu Lys Glu Glu Met
        115                 120                 125

Arg Thr Gln Phe Asn Asp Met Asn Ser Ile Leu Val Thr Ala Ile Pro
    130                 135                 140

Leu Phe Ser Val Gln Asn Tyr Gln Val Pro Phe Leu Ser Val Tyr Val
145                 150                 155                 160

Gln Ala Ala Asn Leu His Leu Ser Val Leu Arg Asp Val Ser Val Phe
                165                 170                 175

Gly Gln Ala Trp Gly Phe Asp Ile Ala Thr Ile Asn Ser Arg Tyr Asn
            180                 185                 190

Asp Leu Thr Arg Leu Ile Pro Ile Tyr Thr Asp Tyr Ala Val Arg Trp
        195                 200                 205

Tyr Asn Thr Gly Leu Asp Arg Leu Pro Arg Thr Gly Gly Leu Arg Asn
    210                 215                 220

Trp Ala Arg Phe Asn Gln Phe Arg Arg Glu Leu Thr Ile Ser Val Leu
225                 230                 235                 240

Asp Ile Ile Ser Phe Phe Arg Asn Tyr Asp Ser Arg Leu Tyr Pro Ile
                245                 250                 255

Pro Thr Ser Ser Gln Leu Thr Arg Glu Val Tyr Thr Asp Pro Val Ile
            260                 265                 270

Asn Ile Thr Asp Tyr Arg Val Gly Pro Ser Phe Glu Asn Ile Glu Asn
        275                 280                 285

Ser Ala Ile Arg Ser Pro His Leu Met Asp Phe Leu Asn Asn Leu Thr
    290                 295                 300

Ile Asp Thr Asp Leu Ile Arg Gly Val His Tyr Trp Ala Gly His Arg
305                 310                 315                 320

Val Thr Ser His Phe Thr Gly Ser Ser Gln Val Ile Thr Thr Pro Gln
                325                 330                 335

Tyr Gly Ile Thr Ala Asn Ala Glu Pro Arg Arg Thr Ile Ala Pro Ser
            340                 345                 350

Thr Phe Pro Gly Leu Asn Leu Phe Tyr Arg Thr Leu Ser Asn Pro Phe
        355                 360                 365

Phe Arg Arg Ser Glu Asn Ile Thr Pro Thr Leu Gly Ile Asn Val Val
    370                 375                 380

Gln Gly Val Gly Phe Ile Gln Pro Asn Asn Ala Glu Val Leu Tyr Arg

385                 390                 395                 400

Ser Arg Gly Thr Val Asp Ser Leu Asn Glu Leu Pro Ile Asp Gly Glu
                405                 410                 415

Asn Ser Leu Val Gly Tyr Ser His Arg Leu Ser His Val Thr Leu Thr
            420                 425                 430

Arg Ser Leu Tyr Asn Thr Asn Ile Thr Ser Leu Pro Thr Phe Val Trp
        435                 440                 445

Thr His His Ser Ala Thr Asn Thr Asn Thr Ile Asn Pro Asp Ile Ile
    450                 455                 460

Thr Gln Ile Pro Leu Val Lys Gly Phe Arg Leu Gly Gly Gly Thr Ser
465                 470                 475                 480

Val Ile Lys Gly Pro Gly Phe Thr Gly Gly Asp Ile Leu Arg Arg Asn
                485                 490                 495

Thr Ile Gly Glu Phe Val Ser Leu Gln Val Asn Ile Asn Ser Pro Ile
            500                 505                 510

Thr Gln Arg Tyr Arg Leu Arg Phe Arg Tyr Ala Ser Ser Arg Asp Ala
        515                 520                 525

Arg Ile Thr Val Ala Ile Gly Gly Gln Ile Arg Val Asp Met Thr Leu
    530                 535                 540

Glu Lys Thr Met Glu Ile Gly Glu Ser Leu Thr Ser Arg Thr Phe Ser
545                 550                 555                 560

Tyr Thr Asn Phe Ser Asn Pro Phe Ser Phe Arg Ala Asn Pro Asp Ile
                565                 570                 575

Ile Arg Ile Ala Glu Glu Leu Pro Ile Arg Gly Gly Glu Leu Tyr Ile
            580                 585                 590

Asp Lys Ile Glu Leu Ile Leu Ala Asp Ala Thr
        595                 600

## Description

### BACKGROUND

Humans grow corn for food and energy applications. Insects eat and damage corn plants and thereby undermine these human efforts. Agricultural insect pests causing major destruction of corn are the fall armyworm (FAW, Spodoptera frugiperda) and European corn borer (ECB, Ostrinia nubilalis).

Current in-plant transgenic control of these pests is achieved through plant expression of a crystal (Cry) delta endotoxin gene coding for the Cry1Fa protein from Bacillus thuringiensis. CrylFa is the protein toxin currently in the Herculex™ brand of Dow AgroSciences transgenic corn seeds (Herculex, Herculex-Extra, and Herculex-RW) that are resistant to FAW and ECB insect pests. This protein works by binding to specific receptor(s) located in the midgut of insects, and forms pores within the gut cells. The formation of these pores prevents insects from regulating osmotic balance which results in their death.

However, some are concerned that insects might be able to develop resistance to the action of CrylFa through genetic alterations of the receptors within their gut that bind CrylFa. Insects that produce receptors with a reduced ability to bind CrylFa can be resistant to the activity of CrylFa, and thus survive on plants that express this protein.

With a single Cry toxin continuously present in the plant during growth conditions, there is concern that insects could develop resistance to the activity of this protein through genetic alterations of the receptor that binds CrylFa toxin in the insect gut. Reductions in toxin binding due to these alterations in the receptor would lead to reduced toxicity of the CrylFa possibly leading to eventual decreased effectiveness of the protein when expressed in a crop.

### BRIEF SUMMARY

The subject invention relates in part to stacking certain Cry genes along with Cry1Fa to result in products that are more durable and less prone towards insects developing resistance towards the activity of any of the toxins (such as Cry1Fa) by itself. Embodiments of the Cry1F stacking partners include Cry2Aa and/or Cry1I - for targeting fall armyworm (FAW; *Spodoptera frugiperda*), and Cry1E - for targeting European cornborer (ECB; *Ostrinia nubilalis*).

### DETAILED DESCRIPTION

The subject invention includes the use of at least one of the stacking partner toxins with CrylFa toxins as a pair. Some preferred pairs for targeting FAW (fall armyworm; *Spodoptera frugiperda*) are a Cry1Fa protein plus a Cry1Ea protein. Some preferred pairs for targeting ECB (European cornborer; *Ostrinia nubilalis*) comprise a Cry1Fa protein plus a Cry1I and/or a Cry2Aa protein.

The subject invention also relates in part to triple stacks or "pyramids" of three (or more) toxins, with Cry1Fa and a stacking partner toxin being the base pair. One preferred pyramid provides two modes of action against two pests - the FAW and the ECB. This type of "2 MOA" pyramid includes: Cry1Fa plus Cry2Aa plus Cry1Ab (a preferred embodiment for controlling ECB), and Cry1Fa plus Cry1Ea (a preferred embodiment for controlling FAW). By "separate modes of action" it is meant that the proteins do not cause cross-resistance with each other.

In some preferred pyramid embodiments, the selected toxins provide three separate modes of action (active ingredients that do not result in cross-resistance) against ECB. Preferred pyramid combinations are Cry1Fa plus a second IRM toxin plus a third IRM toxin. Examples of such are as follows.

ECB pyramids of the subject invention include a Cry1Fa toxin plus a Cry2Aa toxin as the second IRM toxin and a third IRM toxin selected from the group consisting of Cry1Be, Cry1Ab, DIG-3, and Cry1I toxins.

ECB pyramids of the subject invention include a Cry1Fa toxin plus a Cry1I toxin as the second IRM toxin and a third IRM toxin selected from the group consisting of Cry1Ab, Cry1Be, DIG-3, and Cry2Aa toxins.

These various toxins (and others) are listed in the attached Appendix A. Those GENBANK numbers can also be used to obtain the sequences for any of the genes and proteins disclosed or mentioned herein.

Patents can also be used to obtain relevant sequences. For example, U.S. Patent No. 5,188,960 and U.S. Patent No. 5,827,514 describe Cry1Fa core toxin containing proteins suitable for use in carrying out the present invention. U.S. Patent No. 6,218,188 describes plant-optimized DNA sequences encoding CrylFa core toxin-containing proteins that are suitable for use in the present invention. US 2010-00269223 relates to DIG-3 proteins.

The subject invention also relates generally to the use of three insecticidal proteins (*Cry* proteins in some preferred embodiments) that do not compete with each other / do not result in cross-resistance yet are active against a single target pest.

Plants (and acreage planted with such plants) that produce any of the the subject pairs, or three (or more), toxins are included within the scope of the subject invention. Additional toxins/genes can also be added, but these particular triple stacks would, according to the subject invention, advantageously and surprisingly provide three modes of action (non-cross-resistant activity) against FAW and/or ECB. This can help to reduce or eliminate the requirement for refuge acreage (e.g., less than 40%, less than 20%, less than 10%, less than 5%, or even 0% refuge). A field thus planted of over 10 acres is thus included within the subject invention.

The subject polynucleotide(s) are preferably in a genetic construct under control of (operably linked to / comprising) a non-*Bacillus-thuringiensis* promoter. The subject polynucleotides can comprise plant codon usage for enhanced expression in a plant.

To counter act the ability of insects to develop resistance to Cry1Fa, we identified Cry toxins that non-competitively (with Cry1Fa) bind to FAW and/or ECB gut cell preparations. Cry1Fa does not to displace binding of Cry proteins identified herein in the insect gut of FAW and ECB larvae - Cry proteins such as Cry2Aa. The ability of these Cry toxins to be toxic to FAW and/or ECB larvae, yet not fully interact with the same sites as Cry1Fa, shows that their toxicity will not be affected by insects having developed genetic alterations of their Cry1Fa receptor as a mechanism to become resistant to the toxicity of Cry1Fa.

Thus insects having developed resistance to Cry1Fa through a reduction in the ability of its gut receptors to bind Cry1Fa would still be susceptible to the toxicity of Cry2Aa proteins, for example, which bind alternative sites. We have obtained biochemical data that supports this. Having combinations of these proteins expressed in transgenic plants thus provides a useful and valuable mechanism to reduce the probability for the development of insect resistance in the field and thus lead towards a reduction in the requirement for refugia. Other Cry proteins have been studied for their activity against other major insect pests, both sensitive, and those resistant to Cry1Fa (rFAW and rECB). As shown in Table 1, Cry1I and Cry2Aa are active against both resistant and susceptible ECB larvae. Cry1Ea is active against both resistant and susceptible FAW. Binding data for these Cry toxins against these insect pests can be obtained. In any case, the data herein described below shows the toxins interacting at separate target site(s) within the insect gut compared to Cry1Fa and thus would make excellent stacking partners.

Stacking Cry1Fa expressing crops with one or more additional Cry genes, such as those described herein result in an effective management strategy to prevent the ability of insects to develop tolerance to the activity of transgenic plants expressing these protein toxins. Since we show that these Cry proteins interact at different and/or overlapping sites compared to Cry1Fa, if resistance were to occur through alterations in the affinity of the insect gut receptors that bind to the Cry toxins, the alteration would have to occur in at least two different receptors simultaneously to allow the insects to survive on plants expressing the multiple proteins. The probability of this occurring is extremely remote, thus increasing the durability of the transgenic product to ward of insects being able to develop tolerance to the proteins.

We radio-iodinated trypsin truncated forms of the Cry protein toxins and used radioreceptor binding assay techniques to measure their binding interaction with putative receptor proteins located within the insect gut membranes. The gut membranes were prepared as brush border membrane vesicles (BBMV) by the method of Wolfersberger. Iodination of the toxins were conducted using either iodo beads or iodogen treated tubes from Pierce Chemicals. Specific activity of the radiolabeled toxin was approximately 1-4 µCi/µg protein. Binding studies were carried out essentially by the procedures of Liang.

The data presented herein shows the toxins interacting at separate target site within the insect gut compared to Cry1Fa and thus would make excellent stacking partners.

The subject invention can be used with a variety of plants. Examples include corn (maize), soybeans, and cotton.

Genes and toxins useful according to the subject invention include not only the full length sequences disclosed but also fragments of these sequences, variants, mutants, and fusion proteins which retain the characteristic pesticidal activity of the toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins.

As used therein, the boundaries represent approximately 95% (*e.g.* Cry1Fa's), 78% (*e.g.* Cry1F's), and 45% (Cry1's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. These cut offs can also be applied to the core proteins only (for Cry1F and Cry1Fa core proteins, for example). *See* related Crickmore *et al.* website at lifesci.sussex.ac.uk/home/Neil_Crickmore/Bt/.

Fragments and equivalents which retain the pesticidal activity of the exemplified toxins would be within the scope of the subject invention. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. These variant DNA sequences are within the scope of the subject invention. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; IX SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Certain proteins of the subject invention have been specifically exemplified herein. Since these proteins are merely exemplary of the proteins of the subject invention, it should be readily apparent that the subject invention comprises variant or equivalent proteins (and nucleotide sequences coding for equivalent proteins) having the same or similar pesticidal activity of the exemplified protein. Equivalent proteins will have amino acid homology with an exemplified protein. This amino acid homology will typically be greater than 75%, preferably be greater than 90%, and most preferably be greater than 95%. The amino acid homology will be highest in critical regions of the protein which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Following is a listing of examples of amino acids belonging to each class. In some instances, non-conservative substitutions can also be made. The critical factor is that these substitutions must not significantly detract from the biological activity of the protein.

| **Class of Amino Acid** | **Examples of Amino Acids** |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

Insect Resistance Management (IRM) Strategies. Roush *et al*., for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppd1/biopesticides/pips/bt_corn_refuge_2006.htm) publishes the following requirements for providing non-transgenic (*i.e*., *non-B.t*.*)* refuges (a section of non-Bt crops / corn) for use with transgenic crops producing a single Bt protein active against target pests.

"The specific structured requirements for corn borer-protected Bt (Cry1Ab
or Cry IF) corn products are as follows:
Structured refuges: 20% non-Lepidopteran Bt corn refuge in Corn Belt;
   50% non-Lepidopteran Bt refuge in Cotton Belt
Blocks
   Internal (*i.e.,* within the Bt field)
   External (*i.e.,* separate fields within ½ mile (¼ mile if possible) of the Bt field to maximize random mating)
In-field Strips
   Strips must be at least 4 rows wide (preferably 6 rows) to reduce the effects of larval movement"

In addition, the National Corn Growers Association, on their website (ncga.com/insect-resistance-management-fact-sheet-bt-corn) also provides similar guidance regarding the refuge requirements. For example:
"Requirements of the Corn Borer IRM:
- Plant at least 20% of your corn acres to refuge hybrids
- In cotton producing regions, refuge must be 50%
- Must be planted within 1/2 mile of the refuge hybrids
- Refuge can be planted as strips within the Bt field; the refuge strips must be at least 4 rows wide
- Refuge may be treated with conventional pesticides only if economic thresholds are reached for target insect
- Bt-based sprayable insecticides cannot be used on the refuge corn
- Appropriate refuge must be planted on every farm with Bt corn"

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject double or triple stacks or pyramids. For triple stacks with three sites of action against a single target pest, a goal would be zero refuge (or less than 5% refuge, for example). This is particularly true for commercial acreage - of over 10 acres for example.

All patents, patent applications, provisional applications, and publications referred to or cited herein are incorporated by reference in their entirety to the extent they are not inconsistent with the explicit teachings of this specification.

Unless specifically indicated or implied, the terms "a", "an", and "the" signify "at least one" as used herein.

Following are examples that illustrate procedures for practicing the invention. These examples should not be construed as limiting. All percentages are by weight and all solvent mixture proportions are by volume unless otherwise noted. All temperatures are in degrees Celsius.

### REFERENCES

Wolfersberger, M.G., (1993), Preparation and Partial Characterization of Amino Acid Transporting Brush Border Membrane Vesicles from the Larval Midgut of the Gypsy Moth (Lymantria Dispar). Arch. Insect Biochem. Physiol. 24: 139-147.
Liang, Y., Patel, S.S., and Dean, D.H., (1995), Irreversible Binding Kinetics of Bacillus thuringiensis CrylA Delta-Endotoxins to Gypsy Moth Brush Border Membrane Vesicles is Directly Correlated to Toxicity. J. Biol. Chem., 270, 24719-24724.

### Example 1 - Bioassay

The subject Cry proteins were studied for their activity against other major insect pests, both sensitive, and those resistant to CrylFa (rFAW and rECB). As shown in Table 1, Cry1I and Cry2Aa are active against both resistant and susceptible ECB larvae. CrylEa is active against both resistant and susceptible FAW. (For a general discussion of this pest, *see e.g.* Tabashnik, PNAS (2008), vol. 105 no. 49, 19029-19030.)

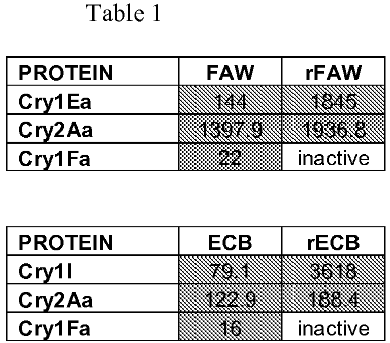

Appendix A

List of delta-endotoxins – from Crickmore et al. website (cited in application)

Accession Number is to NCBI entry (if available)

Name | Acc No. | Authors | Year | Source Strain | Comment
Cry1Aa1 | AAA22353 | Schnepf et al | 1985 | Bt kurstaki HD1 |
Cry1Aa2 | AAA22552 | Shibano et al | 1985 | Bt sotto |
Cry1Aa3 | BAA00257 | Shimizu et al | 1988 | Bt aizawai IPL7 |
Cry1Aa4 | CAA31886 | Masson et al | 1989 | Bt entomocidus |
Cry1Aa5 | BAA04468 | Udayasuriyan et al | 1994 | Bt Fu-2-7 |
Cry1Aa6 | AAA86265 | Masson et al | 1994 | Bt kurstaki NRD-
12 |
Cry1Aa7 | AAD46139 | Osman et al | 1999 | Bt C12 |
Cry1Aa8 | I26149 | Liu | 1996 | | DNA sequence only
Cry1Aa9 | BAA77213 | Nagamatsu et al | 1999 | Bt dendrolimus
T84A1 |
Cry1Aa10 | AAD55382 | Hou and Chen | 1999 | Bt kurstaki HD-1-
02 |
Cry1Aa11 | CAA70856 | Tounsi et al | 1999 | Bt kurstaki |
Cry1Aa12 | AAP80146 | Yao et al | 2001 | Bt Ly30 |
Cry1Aa13 | AAM44305 | Zhong et al | 2002 | Bt sotto |
Cry1Aa14 | AAP40639 | Ren et al | 2002 | unpublished |
Cry1Aa15 | AAY66993 | Sauka et al | 2005 | Bt INTA Mol-12 |
Cry1Ab1 | AAA22330 | Wabiko et al | 1986 | Bt berliner 1715 |
Cry1Ab2 | AAA22613 | Thorne et al | 1986 | Bt kurstaki |
Cry1Ab3 | AAA22561 | Geiser et al | 1986 | Bt kurstaki HD1 |
Cry1Ab4 | BAA00071 | Kondo et al | 1987 | Bt kurstaki HD1 |
Cry1Ab5 | CAA28405 | Hofte et al | 1986 | Bt berliner 1715 |
Cry1Ab6 | AAA22420 | Hefford et al | 1987 | Bt kurstaki NRD-
12 |
Cry1Ab7 | CAA31620 | Haider & Ellar | 1988 | Bt aizawai IC1 |
Cry1Ab8 | AAA22551 | Oeda et al | 1987 | Bt aizawai IPL7 |
Cry1Ab9 | CAA38701 | Chak & Jen | 1993 | Bt aizawai HD133 |
Cry1Ab10 | A29125 | Fischhoff et al | 1987 | Bt kurstaki HD1 |
Cry1Ab11 | I12419 | Ely & Tippett | 1995 | Bt A20 | DNA sequence only
Cry1Ab12 | AAC64003 | Silva-Werneck et al | 1998 | Bt kurstaki S93 |

Cry1Ab13 | AAN76494 | Tan et al | 2002 | Bt c005
Cry1Ab14 | AAG16877 | Meza-Basso &
Theoduloz | 2000 | Native Chilean Bt
Cry1Ab15 | AAO13302 | Li et al | 2001 | Bt B-Hm-16
Cry1Ab16 | AAK55546 | Yu et al | 2002 | Bt AC-11
Cry1Ab17 | AAT46415 | Huang et al | 2004 | Bt WB9
Cry1Ab18 | AAQ88259 | Stobdan et al | 2004 | Bt
Cry1Ab19 | AAW31761 | Zhong et al | 2005 | Bt X-2
Cry1Ab20 | ABB72460 | Liu et al | 2006 | BtC008
Cry1Ab21 | ABS18384 | Swiecicka et al | 2007 | Bt IS5056
Cry1Ab22 | ABW87320 | Wu and Feng | 2008 | BtS2491Ab
Cry1Ab-
like | AAK14336 | Nagarathinam et al | 2001 | Bt kunthala RX24 | uncertain sequence
Cry1Ab-
like | AAK14337 | Nagarathinam et al | 2001 | Bt kunthala RX28 | uncertain sequence
Cry1Ab-
like | AAK14338 | Nagarathinam et al | 2001 | Bt kunthala RX27 | uncertain sequence
Cry1Ab-
like | ABG88858 | Lin et al | 2006 | Bt ly4a3 | insufficient sequence
Cry1Ac1 | AAA22331 | Adang et al | 1985 | Bt kurstaki HD73
Cry1Ac2 | AAA22338 | Von Tersch et al | 1991 | Bt kenyae
Cry1Ac3 | CAA38098 | Dardenne et al | 1990 | Bt BTS89A
Cry1Ac4 | AAA73077 | Feitelson | 1991 | Bt kurstaki
PS85A1
Cry1Ac5 | AAA22339 | Feitelson | 1992 | Bt kurstaki
PS81GG
Cry1Ac6 | AAA86266 | Masson et al | 1994 | Bt kurstaki NRD-
12
Cry1Ac7 | AAB46989 | Herrera et al | 1994 | Bt kurstaki HD73
Cry1Ac8 | AAC44841 | Omolo et al | 1997 | Bt kurstaki HD73
Cry1Ac9 | AAB49768 | Gleave et al | 1992 | Bt DSIR732
Cry1Ac10 | CAA05505 | Sun | 1997 | Bt kurstaki YBT-
1520
Cry1Ac11 | CAA10270 | Makhdoom &
Riazuddin | 1998
Cry1Ac12 | I12418 | Ely & Tippett | 1995 | Bt A20 | DNA sequence only
Cry1Ac13 | AAD38701 | Qiao et al | 1999 | Bt kurstaki HD1
Cry1Ac14 | AAQ06607 | Yao et al | 2002 | Bt Ly30
Cry1Ac15 | AAN07788 | Tzeng et al | 2001 | Bt from Taiwan
Cry1Ac16 | AAU87037 | Zhao et al | 2005 | Bt H3

Cry1Ac17 | AAX18704 | Hire et al | 2005 | Bt kenyae HD549 |
Cry1Ac18 | AAY88347 | Kaur & Allam | 2005 | Bt SK-729 |
Cry1Ac19 | ABD37053 | Gao et al | 2005 | Bt C-33 |
Cry1Ac20 | ABB89046 | Tan et al | 2005 | |
Cry1Ac21 | AAY66992 | Sauka et al | 2005 | INTA Mol-12 |
Cry1Ac22 | ABZ01836 | Zhang & Fang | 2008 | Bt W015-1 |
Cry1Ac23 | CAQ30431 | Kashyap et al | 2008 | Bt |
Cry1Ac24 | ABL01535 | Arango et al | 2008 | Bt 146-158-01 |
Cry1Ac25 | FJ513324 | Guan Peng et al | 2008 | Bt Tm37-6 | No NCBI link July 09
Cry1Ac26 | FJ617446 | Guan Peng et al | 2009 | Bt Tm41-4 | No NCBI link July 09
Cry1Ac27 | FJ617447 | Guan Peng et al | 2009 | Bt Tm44-1B | No NCBI link July 09
Cry1Ac28 | ACM90319 | Li et al | 2009 | Bt Q-12 |
Cry1Ad1 | AAA22340 | Feitelson | 1993 | Bt aizawai PS81I |
Cry1Ad2 | CAA01880 | Anonymous | 1995 | Bt PS81RR1 |
Cry1Ae1 | AAA22410 | Lee & Aronson | 1991 | Bt alesti |
Cry1Af1 | AAB82749 | Kang et al | 1997 | Bt NT0423 |
Cry1Ag1 | AAD46137 | Mustafa | 1999 | |
Cry1Ah1 | AAQ14326 | Tan et al | 2000 | |
Cry1Ah2 | ABB76664 | Qi et al | 2005 | Bt alesti |
Cry1Ai1 | AAO39719 | Wang et al | 2002 | |
Cry1A-
like | AAK14339 | Nagarathinam et al | 2001 | Bt kunthala nags3 | uncertain sequence
Cry1Ba1 | CAA29898 | Brizzard & Whiteley | 1988 | Bt thuringiensis
HD2 |
Cry1Ba2 | CAA65003 | Soetaert | 1996 | Bt entomocidus
HD110 |
Cry1Ba3 | AAK63251 | Zhang et al | 2001 | |
Cry1Ba4 | AAK51084 | Nathan et al | 2001 | Bt entomocidus
HD9 |
Cry1Ba5 | ABO20894 | Song et al | 2007 | Bt sfw-12 |
Cry1Ba6 | ABL60921 | Martins et al | 2006 | Bt S601 |
Cry1Bb1 | AAA22344 | Donovan et al | 1994 | Bt EG5847 |
Cry1Bc1 | CAA86568 | Bishop et al | 1994 | Bt morrisoni |
Cry1Bd1 | AAD10292 | Kuo et al | 2000 | Bt wuhanensis
HD525 |
Cry1Bd2 | AAM93496 | Isakova et al | 2002 | Bt 834 |
Cry1Be1 | AAC32850 | Payne et al | 1998 | Bt PS158C2 |
Cry1Be2 | AAQ52387 | Baum et al | 2003 | |
Cry1Be3 | FJ716102 | Xiaodong Sun et al | 2009 | Bt | No NCBI link July 09
Cry1Bf1 | CAC50778 | Arnaut et al | 2001 | |

Cry1Bf2 | AAQ52380 | Baum et al | 2003 | |
Cry1Bg1 | AAO39720 | Wang et al | 2002 | |
Cry1Ca1 | CAA30396 | Honee et al | 1988 | Bt entomocidus 60.5 |
Cry1Ca2 | CAA31951 | Sanchis et al | 1989 | Bt aizawai 7.29 |
Cry1Ca3 | AAA22343 | Feitelson | 1993 | Bt aizawai PS81I |
Cry1Ca4 | CAA01886 | Van Mellaert et al | 1990 | Bt entomocidus HD110 |
Cry1Ca5 | CAA65457 | Strizhov | 1996 | Bt aizawai 7.29 |
Cry1Ca6 | AAF37224 | Yu et al | 2000 | Bt AF-2 |
Cry1Ca7 | AAG50438 | Aixing et al | 2000 | Bt J8 |
Cry1Ca8 | AAM00264 | Chen et al | 2001 | Bt c002 |
Cry1Ca9 | AAL79362 | Kao et al | 2003 | Bt G10-01A |
Cry1Ca10 | AAN16462 | Lin et al | 2003 | Bt E05-20a |
Cry1Ca11 | AAX53094 | Cai et al | 2005 | Bt C-33 |
Cry1Cb1 | M97880 | Kalman et al | 1993 | Bt galleriae HD29 | DNA sequence only
Cry1Cb2 | AAG35409 | Song et al | 2000 | Bt c001 |
Cry1Cb3 | ACD50894 | Huang et al | 2008 | Bt 087 |
Cry1Cb-like | AAX63901 | Thammasittirong et al | 2005 | Bt TA476-1 | insufficient sequence
Cry1Da1 | CAA38099 | Hofte et al | 1990 | Bt aizawai HD68 |
Cry1Da2 | I76415 | Payne & Sick | 1997 | | DNA sequence only
Cry1Db1 | CAA80234 | Lambert | 1993 | Bt BTS00349A |
Cry1Db2 | AAK48937 | Li et al | 2001 | Bt B-Pr-88 |
Cry1Dc1 | ABK35074 | Lertwiriyawong et al | 2006 | Bt JC291 |
Cry1Ea1 | CAA37933 | Visser et al | 1990 | Bt kenyae 4F1 |
Cry1Ea2 | CAA39609 | Bosse et al | 1990 | Bt kenyae |
Cry1Ea3 | AAA22345 | Feitelson | 1991 | Bt kenyae PS81F |
Cry1Ea4 | AAD04732 | Barboza-Corona et al | 1998 | Bt kenyae LBIT-147 |
Cry1Ea5 | A15535 | Botterman et al | 1994 | | DNA sequence only
Cry1Ea6 | AAL50330 | Sun et al | 1999 | Bt YBT-032 |
Cry1Ea7 | AAW72936 | Huehne et al | 2005 | Bt JC190 |
Cry1Ea8 | ABX11258 | Huang et al | 2007 | Bt HZM2 |
Cry1Eb1 | AAA22346 | Feitelson | 1993 | Bt aizawai PS81A2 |
Cry1Fa1 | AAA22348 | Chambers et al | 1991 | Bt aizawai EG6346 |
Cry1Fa2 | AAA22347 | Feitelson | 1993 | Bt aizawai PS81I |
Cry1Fb1 | CAA80235 | Lambert | 1993 | Bt BTS00349A |

Cry1Fb2 | BAA25298 | Masuda & Asano | 1998 | Bt morrisoni INA67
Cry1Fb3 | AAF21767 | Song et al | 1998 | Bt morrisoni
Cry1Fb4 | AAC10641 | Payne et al | 1997 |
Cry1Fb5 | AAO13295 | Li et al | 2001 | Bt B-Pr-88
Cry1Fb6 | ACD50892 | Huang et al | 2008 | Bt 012
Cry1Fb7 | ACD50893 | Huang et al | 2008 | Bt 087
Cry1Ga1 | CAA80233 | Lambert | 1993 | Bt BTS0349A
Cry1Ga2 | CAA70506 | Shevelev et al | 1997 | Bt wuhanensis
Cry1Gb1 | AAD10291 | Kuo & Chak | 1999 | Bt wuhanensis HD525
Cry1Gb2 | AAO13756 | Li et al | 2000 | Bt B-Pr-88
Cry1Gc | AAQ52381 | Baum et al | 2003 |
Cry1Ha1 | CAA80236 | Lambert | 1993 | Bt BTS02069AA
Cry1Hb1 | AAA79694 | Koo et al | 1995 | Bt morrisoni BF190
Cry1H-like | AAF01213 | Srifah et al | 1999 | Bt JC291 | insufficient sequence
Cry1Ia1 | CAA44633 | Tailor et al | 1992 | Bt kurstaki
Cry1Ia2 | AAA22354 | Gleave et al | 1993 | Bt kurstaki
Cry1Ia3 | AAC36999 | Shin et al | 1995 | Bt kurstaki HD1
Cry1Ia4 | AAB00958 | Kostichka et al | 1996 | Bt AB88
Cry1Ia5 | CAA70124 | Selvapandiyan | 1996 | Bt 61
Cry1Ia6 | AAC26910 | Zhong et al | 1998 | Bt kurstaki S101
Cry1Ia7 | AAM73516 | Porcar et al | 2000 | Bt
Cry1Ia8 | AAK66742 | Song et al | 2001 |
Cry1Ia9 | AAQ08616 | Yao et al | 2002 | Bt Ly30
Cry1Ia10 | AAP86782 | Espindola et al | 2003 | Bt thuringiensis
Cry1Ia11 | CAC85964 | Tounsi et al | 2003 | Bt kurstaki BNS3
Cry1Ia12 | AAV53390 | Grossi de Sa et al | 2005 | Bt
Cry1Ia13 | ABF83202 | Martins et al | 2006 | Bt
Cry1Ia14 | ACG63871 | Liu & Guo | 2008 | Bt11
Cry1Ia15 | FJ617445 | Guan Peng et al | 2009 | Bt E-1B | No NCBI link July 2009
Cry1Ia16 | FJ617448 | Guan Peng et al | 2009 | Bt E-1A | No NCBI link July 2009
Cry1Ib1 | AAA82114 | Shin et al | 1995 | Bt entomocidus BP465
Cry1Ib2 | ABW88019 | Guan et al | 2007 | Bt PP61
Cry1Ib3 | ACD75515 | Liu & Guo | 2008 | Bt GS8

Cry1Ic1 | AAC62933 | Osman et al | 1998 | Bt C18 |
Cry1Ic2 | AAE71691 | Osman et al | 2001 | |
Cry1Id1 | AAD44366 | Choi | 2000 | |
Cry1Ie1 | AAG43526 | Song et al | 2000 | Bt BTC007 |
Cry1If1 | AAQ52382 | Baum et al | 2003 | |
Cry1I-like | AAC31094 | Payne et al | 1998 | | insufficient sequence
Cry1I-like | ABG88859 | Lin & Fang | 2006 | Bt ly4a3 | insufficient sequence
Cry1Ja1 | AAA22341 | Donovan | 1994 | Bt EG5847 |
Cry1Jb1 | AAA98959 | Von Tersch &
Gonzalez | 1994 | Bt EG5092 |
Cry1Jc1 | AAC31092 | Payne et al | 1998 | |
Cry1Jc2 | AAQ52372 | Baum et al | 2003 | |
Cry1Jd1 | CAC50779 | Arnaut et al | 2001 | Bt |
Cry1Ka1 | AAB00376 | Koo et al | 1995 | Bt morrisoni
BF190 |
Cry1La1 | AAS60191 | Je et al | 2004 | Bt kurstaki K1 |
Cry1-like | AAC31091 | Payne et al | 1998 | | insufficient sequence
Cry2Aa1 | AAA22335 | Donovan et al | 1989 | Bt kurstaki |
Cry2Aa2 | AAA83516 | Widner & Whiteley | 1989 | Bt kurstaki HD1 |
Cry2Aa3 | D86064 | Sasaki et al | 1997 | Bt sotto | DNA sequence only
Cry2Aa4 | AAC04867 | Misra et al | 1998 | Bt kenyae HD549 |
Cry2Aa5 | CAA10671 | Yu & Pang | 1999 | Bt SL39 |
Cry2Aa6 | CAA10672 | Yu & Pang | 1999 | Bt YZ71 |
Cry2Aa7 | CAA10670 | Yu & Pang | 1999 | Bt CY29 |
Cry2Aa8 | AAO13734 | Wei et al | 2000 | Bt Dongbei 66 |
Cry2Aa9 | AAO13750 | Zhang et al | 2000 | |
Cry2Aa10 | AAQ04263 | Yao et al | 2001 | |
Cry2Aa11 | AAQ52384 | Baum et al | 2003 | |
Cry2Aa12 | ABI83671 | Tan et al | 2006 | Bt Rpp39 |
Cry2Aa13 | ABL01536 | Arango et al | 2008 | Bt 146-158-01 |
Cry2Aa14 | ACF04939 | Hire et al | 2008 | Bt HD-550 |
Cry2Ab1 | AAA22342 | Widner & Whiteley | 1989 | Bt kurstaki HD1 |
Cry2Ab2 | CAA39075 | Dankocsik et al | 1990 | Bt kurstaki HD1 |
Cry2Ab3 | AAG36762 | Chen et al | 1999 | Bt BTC002 |
Cry2Ab4 | AAO13296 | Li et al | 2001 | Bt B-Pr-88 |
Cry2Ab5 | AAQ04609 | Yao et al | 2001 | Bt ly30 |
Cry2Ab6 | AAP59457 | Wang et al | 2003 | Bt WZ-7 |
Cry2Ab7 | AAZ66347 | Udayasuriyan et al | 2005 | Bt 14-1 |
Cry2Ab8 | ABC95996 | Huang et al | 2006 | Bt WB2 |

Cry2Ab9 | ABC74968 | Zhang et al | 2005 | Bt LLB6
Cry2Ab10 | EF157306 | Lin et al | 2006 | Bt LyD
Cry2Ab11 | CAM84575 | Saleem et al | 2007 | Bt CMBL-BT1
Cry2Ab12 | ABM21764 | Lin et al | 2007 | Bt LyD
Cry2Ab13 | ACG76120 | Zhu et al | 2008 | Bt ywc5-4
Cry2Ab14 | ACG76121 | Zhu et al | 2008 | Bt Bts
Cry2Ac1 | CAA40536 | Aronson | 1991 | Bt shanghai S1
Cry2Ac2 | AAG35410 | Song et al | 2000
Cry2Ac3 | AAQ52385 | Baum et al | 2003
Cry2Ac4 | ABC95997 | Huang et al | 2006 | Bt WB9
Cry2Ac5 | ABC74969 | Zhang et al | 2005
Cry2Ac6 | ABC74793 | Xia et al | 2006 | Bt wuhanensis
Cry2Ac7 | CAL18690 | Saleem et al | 2008 | Bt SBSBT-1
Cry2Ac8 | CAM09325 | Saleem et al | 2007 | Bt CMBL-BT1
Cry2Ac9 | CAM09326 | Saleem et al | 2007 | Bt CMBL-BT2
Cry2Ac10 | ABN15104 | Bai et al | 2007 | Bt QCL-1
Cry2Ac11 | CAM83895 | Saleem et al | 2007 | Bt HD29
Cry2Ac12 | CAM83896 | Saleem et al | 2007 | Bt CMBL-BT3
Cry2Ad1 | AAF09583 | Choi et al | 1999 | Bt BR30
Cry2Ad2 | ABC86927 | Huang et al | 2006 | Bt WB10
Cry2Ad3 | CAK29504 | Saleem et al | 2006 | Bt 5_2AcT(1)
Cry2Ad4 | CAM32331 | Saleem et al | 2007 | Bt CMBL-BT2
Cry2Ad5 | CAO78739 | Saleem et al | 2007 | Bt HD29
Cry2Ae1 | AAQ52362 | Baum et al | 2003
Cry2Af1 | ABO30519 | Beard et al | 2007 | Bt C81
Cry2Ag | ACH91610 | Zhu et al | 2008 | Bt JF19-2
Cry2Ah | EU939453 | Zhang et al | 2008 | Bt | No NCBI link July 09
Cry2Ah2 | ACL80665 | Zhang et al | 2009 | Bt BRC-ZQL3
Cry2Ai | FJ788388 | Udayasuriyan et al | 2009 | Bt | No NCBI link July 09
Cry3Aa1 | AAA22336 | Herrnstadt et al | 1987 | Bt san diego
Cry3Aa2 | AAA22541 | Sekar et al | 1987 | Bt tenebrionis
Cry3Aa3 | CAA68482 | Hofte et al | 1987
Cry3Aa4 | AAA22542 | McPherson et al | 1988 | Bt tenebrionis
Cry3Aa5 | AAA50255 | Donovan et al | 1988 | Bt morrisoni
EG2158
Cry3Aa6 | AAC43266 | Adams et al | 1994 | Bt tenebrionis
Cry3Aa7 | CAB41411 | Zhang et al | 1999 | Bt 22
Cry3Aa8 | AAS79487 | Gao and Cai | 2004 | Bt YM-03
Cry3Aa9 | AAW05659 | Bulla and Candas | 2004 | Bt UTD-001

Cry3Aa10 | AAU29411 | Chen et al | 2004 | Bt 886 |
Cry3Aa11 | AAW82872 | Kurt et al | 2005 | Bt tenebrionis
Mm2 |
Cry3Aa12 | ABY49136 | Sezen et al | 2008 | Bt tenebrionis |
Cry3Ba1 | CAA34983 | Sick et al | 1990 | Bt tolworthi 43F |
Cry3Ba2 | CAA00645 | Peferoen et al | 1990 | Bt PGSI208 |
Cry3Bb1 | AAA22334 | Donovan et al | 1992 | Bt EG4961 |
Cry3Bb2 | AAA74198 | Donovan et al | 1995 | Bt EG5144 |
Cry3Bb3 | I15475 | Peferoen et al | 1995 | | DNA sequence only
Cry3Ca1 | CAA42469 | Lambert et al | 1992 | Bt kurstaki
BtI109P |
Cry4Aa1 | CAA68485 | Ward & Ellar | 1987 | Bt israelensis |
Cry4Aa2 | BAA00179 | Sen et al | 1988 | Bt israelensis
HD522 |
Cry4Aa3 | CAD30148 | Berry et al | 2002 | Bt israelensis |
Cry4A-
like | AAY96321 | Mahalakshmi et al | 2005 | Bt LDC-9 | insufficient sequence
Cry4Ba1 | CAA30312 | Chungjatpornchai et
al | 1988 | Bt israelensis
4Q2-72 |
Cry4Ba2 | CAA30114 | Tungpradubkul et al | 1988 | Bt israelensis |
Cry4Ba3 | AAA22337 | Yamamoto et al | 1988 | Bt israelensis |
Cry4Ba4 | BAA00178 | Sen et al | 1988 | Bt israelensis
HD522 |
Cry4Ba5 | CAD30095 | Berry et al | 2002 | Bt israelensis |
Cry4Ba-
like | ABC47686 | Mahalakshmi et al | 2005 | Bt LDC-9 | insufficient sequence
Cry4Ca1 | EU646202 | Shu et al | 2008 | | No NCBI link July 09
Cry4Cb1 | FJ403208 | Jun & Furong | 2008 | Bt HS18-1 | No NCBI link July 09
Cry4Cb2 | FJ597622 | Jun & Furong | 2008 | Bt Ywc2-8 | No NCBI link July 09
Cry4Cc1 | FJ403207 | Jun & Furong | 2008 | Bt MC28 | No NCBI link July 09
Cry5Aa1 | AAA67694 | Narva et al | 1994 | Bt darmstadiensis
PS17 |
Cry5Ab1 | AAA67693 | Narva et al | 1991 | Bt darmstadiensis
PS17 |
Cry5Ac1 | I34543 | Payne et al | 1997 | | DNA sequence only
Cry5Ad1 | ABQ82087 | Lenane et al | 2007 | Bt L366 |
Cry5Ba1 | AAA68598 | Foncerrada & Narva | 1997 | Bt PS86Q3 |
Cry5Ba2 | ABW88932 | Guo et al | 2008 | YBT 1518 |
Cry6Aa1 | AAA22357 | Narva et al | 1993 | Bt PS52A1 |
Cry6Aa2 | AAM46849 | Bai et al | 2001 | YBT 1518 |

Cry6Aa3 | ABH03377 | Jia et al | 2006 | Bt 96418 |
Cry6Ba1 | AAA22358 | Narva et al | 1991 | Bt PS69D1 |
Cry7Aa1 | AAA22351 | Lambert et al | 1992 | Bt galleriae PGSI245 |
Cry7Ab1 | AAA21120 | Narva & Fu | 1994 | Bt dakota HD511 |
Cry7Ab2 | AAA21121 | Narva & Fu | 1994 | Bt kumamotoensis 867 |
Cry7Ab3 | ABX24522 | Song et al | 2008 | Bt WZ-9 |
Cry7Ab4 | EU380678 | Shu et al | 2008 | Bt | No NCBI link July 09
Cry7Ab5 | ABX79555 | Aguirre-Arzola et al | 2008 | Bt monterrey GM-33 |
Cry7Ab6 | ACI44005 | Deng et al | 2008 | Bt HQ122 |
Cry7Ab7 | FJ940776 | Wang et al | 2009 | | No NCBI link Sept 09
Cry7Ab8 | GU145299 | Feng Jing | 2009 | | No NCBI link Nov 09
Cry7Ba1 | ABB70817 | Zhang et al | 2006 | Bt huazhongensis |
Cry7Ca1 | ABR67863 | Gao et al | 2007 | Bt BTH-13 |
Cry7Da1 | ACQ99547 | Yi et al | 2009 | Bt LH-2 |
Cry8Aa1 | AAA21117 | Narva & Fu | 1992 | Bt kumamotoensis |
Cry8Ab1 | EU044830 | Cheng et al | 2007 | Bt B-JJX | No NCBI link July 09
Cry8Ba1 | AAA21118 | Narva & Fu | 1993 | Bt kumamotoensis |
Cry8Bb1 | CAD57542 | Abad et al | 2002 | |
Cry8Bc1 | CAD57543 | Abad et al | 2002 | |
Cry8Ca1 | AAA21119 | Sato et al. | 1995 | Bt japonensis Buibui |
Cry8Ca2 | AAR98783 | Shu et al | 2004 | Bt HBF-1 |
Cry8Ca3 | EU625349 | Du et al | 2008 | Bt FTL-23 | No NCBI link July 09
Cry8Da1 | BAC07226 | Asano et al | 2002 | Bt galleriae |
Cry8Da2 | BD133574 | Asano et al | 2002 | Bt | DNA sequence only
Cry8Da3 | BD133575 | Asano et al | 2002 | Bt | DNA sequence only
Cry8Db1 | BAF93483 | Yamaguchi et al | 2007 | Bt BBT2-5 |
Cry8Ea1 | AAQ73470 | Fuping et al | 2003 | Bt 185 |
Cry8Ea2 | EU047597 | Liu et al | 2007 | Bt B-DLL | No NCBI link July 09
Cry8Fa1 | AAT48690 | Shu et al | 2004 | Bt 185 | also AAW81032
Cry8Ga1 | AAT46073 | Shu et al | 2004 | Bt HBF-18 |
Cry8Ga2 | ABC42043 | Yan et al | 2008 | Bt 145 |
Cry8Ga3 | FJ198072 | Xiaodong et al | 2008 | Bt FCD114 | No NCBI link July 09
Cry8Ha1 | EF465532 | Fuping et al | 2006 | Bt 185 | No NCBI link July 09
Cry8Ia1 | EU381044 | Yan et al | 2008 | Bt su4 | No NCBI link July 09
Cry8Ja1 | EU625348 | Du et al | 2008 | Bt FPT-2 | No NCBI link July 09
Cry8Ka1 | FJ422558 | Quezado et al | 2008 | | No NCBI link July 09

Cry8Ka2 | ACN87262 | Noguera & Ibarra | 2009 | Bt kenyae |
Cry8-like | FJ770571 | Noguera & Ibarra | 2009 | Bt canadensis | DNA sequence only
Cry8-like | ABS53003 | Mangena et al | 2007 | Bt |
Cry9Aa1 | CAA41122 | Shevelev et al | 1991 | Bt galleriae |
Cry9Aa2 | CAA41425 | Gleave et al | 1992 | Bt DSIR517 |
Cry9Aa3 | GQ249293 | Su et al | 2009 | Bt SC5(D2) | No NCBI link July 09
Cry9Aa4 | GQ249294 | Su et al | 2009 | Bt T03C001 | No NCBI link July 09
Cry9Aa
like | AAQ52376 | Baum et al | 2003 | | incomplete sequence
Cry9Ba1 | CAA52927 | Shevelev et al | 1993 | Bt galleriae |
Cry9Bb1 | AAV28716 | Silva-Werneck et al | 2004 | Bt japonensis |
Cry9Ca1 | CAA85764 | Lambert et al | 1996 | Bt tolworthi |
Cry9Ca2 | AAQ52375 | Baum et al | 2003 | |
Cry9Da1 | BAA19948 | Asano | 1997 | Bt japonensis
N141 |
Cry9Da2 | AAB97923 | Wasano & Ohba | 1998 | Bt japonensis |
Cry9Da3 | GQ249295 | Su et al | 2009 | Bt T03B001 | No NCBI link July 09
Cry9Da4 | GQ249297 | Su et al | 2009 | Bt T03B001 | No NCBI link July 09
Cry9Db1 | AAX78439 | Flannagan & Abad | 2005 | Bt kurstaki
DP1019 |
Cry9Ea1 | BAA34908 | Midoh & Oyama | 1998 | Bt aizawai SSK-
10 |
Cry9Ea2 | AAO12908 | Li et al | 2001 | Bt B-Hm-16 |
Cry9Ea3 | ABM21765 | Lin et al | 2006 | Bt lyA |
Cry9Ea4 | ACE88267 | Zhu et al | 2008 | Bt ywc5-4 |
Cry9Ea5 | ACF04743 | Zhu et al | 2008 | Bts |
Cry9Ea6 | ACG63872 | Liu & Guo | 2008 | Bt 11 |
Cry9Ea7 | FJ380927 | Sun et al | 2008 | | No NCBI link July 09
Cry9Ea8 | GQ249292 | Su et al | 2009 | GQ249292 | No NCBI link July 09
Cry9Eb1 | CAC50780 | Arnaut et al | 2001 | |
Cry9Eb2 | GQ249298 | Su et al | 2009 | Bt T03B001 | No NCBI link July 09
Cry9Ec1 | AAC63366 | Wasano et al | 2003 | Bt galleriae |
Cry9Ed1 | AAX78440 | Flannagan & Abad | 2005 | Bt kurstaki
DP1019 |
Cry9Ee1 | GQ249296 | Su et al | 2009 | Bt T03B001 | No NCBI link Aug 09
Cry9-like | AAC63366 | Wasano et al | 1998 | Bt galleriae | insufficient sequence
Cry10Aa1 | AAA22614 | Thorne et al | 1986 | Bt israelensis |
Cry10Aa2 | E00614 | Aran & Toomasu | 1996 | Bt israelensis
ONR-60A | DNA sequence only
Cry10Aa3 | CAD30098 | Berry et al | 2002 | Bt israelensis |

Cry10A-like | DQ167578 | Mahalakshmi et al | 2006 | Bt LDC-9 | incomplete sequence
Cry11Aa1 | AAA22352 | Donovan et al | 1988 | Bt israelensis |
Cry11Aa2 | AAA22611 | Adams et al | 1989 | Bt israelensis |
Cry11Aa3 | CAD30081 | Berry et al | 2002 | Bt israelensis |
Cry11Aa-like | DQ166531 | Mahalakshmi et al | 2007 | Bt LDC-9 | incomplete sequence
Cry11Ba1 | CAA60504 | Delecluse et al | 1995 | Bt jegathesan 367 |
Cry11Bb1 | AAC97162 | Orduz et al | 1998 | Bt medellin |
Cry12Aa1 | AAA22355 | Narva et al | 1991 | Bt PS33F2 |
Cry13Aa1 | AAA22356 | Narva et al | 1992 | Bt PS63B |
Cry14Aa1 | AAA21516 | Narva et al | 1994 | Bt sotto PS80JJ1 |
Cry15Aa1 | AAA22333 | Brown & Whiteley | 1992 | Bt thompsoni |
Cry16Aa1 | CAA63860 | Barloy et al | 1996 | Cb malaysia CH18 |
Cry17Aa1 | CAA67841 | Barloy et al | 1998 | Cb malaysia CH18 |
Cry18Aa1 | CAA67506 | Zhang et al | 1997 | Paenibacillus popilliae |
Cry18Ba1 | AAF89667 | Patel et al | 1999 | Paenibacillus popilliae |
Cry18Ca1 | AAF89668 | Patel et al | 1999 | Paenibacillus popilliae |
Cry19Aa1 | CAA68875 | Rosso & Delecluse | 1996 | Bt jegathesan 367 |
Cry19Ba1 | BAA32397 | Hwang et al | 1998 | Bt higo |
Cry20Aa1 | AAB93476 | Lee & Gill | 1997 | Bt fukuokaensis |
Cry20Ba1 | ACS93601 | Noguera & Ibarra | 2009 | Bt higo LBIT-976 |
Cry20-like | GQ144333 | Yi et al | 2009 | Bt Y-5 | DNA sequence only
Cry21Aa1 | I32932 | Payne et al | 1996 | | DNA sequence only
Cry21Aa2 | I66477 | Feitelson | 1997 | | DNA sequence only
Cry21Ba1 | BAC06484 | Sato & Asano | 2002 | Bt roskildiensis |
Cry22Aa1 | I34547 | Payne et al | 1997 | | DNA sequence only
Cry22Aa2 | CAD43579 | Isaac et al | 2002 | Bt |
Cry22Aa3 | ACD93211 | Du et al | 2008 | Bt FZ-4 |
Cry22Ab1 | AAK50456 | Baum et al | 2000 | Bt EG4140 |
Cry22Ab2 | CAD43577 | Isaac et al | 2002 | Bt |
Cry22Ba1 | CAD43578 | Isaac et al | 2002 | Bt |
Cry23Aa1 | AAF76375 | Donovan et al | 2000 | Bt | Binary with Cry37Aa1
Cry24Aa1 | AAC61891 | Kawalek and Gill | 1998 | Bt jegathesan |
Cry24Ba1 | BAD32657 | Ohgushi et al | 2004 | Bt sotto |
Cry24Ca1 | CAJ43600 | Beron & Salerno | 2005 | Bt FCC-41 |
Cry25Aa1 | AAC61892 | Kawalek and Gill | 1998 | Bt jegathesan |

Cry26Aa1 | AAD25075 | Wojciechowska et al | 1999 | Bt finitimus B-1166
Cry27Aa1 | BAA82796 | Saitoh | 1999 | Bt higo
Cry28Aa1 | AAD24189 | Wojciechowska et al | 1999 | Bt finitimus B-1161
Cry28Aa2 | AAG00235 | Moore and Debro | 2000 | Bt finitimus
Cry29Aa1 | CAC80985 | Delecluse et al | 2000 | Bt medellin
Cry30Aa1 | CAC80986 | Delecluse et al | 2000 | Bt medellin
Cry30Ba1 | BAD00052 | Ito et al | 2003 | Bt entomocidus
Cry30Ca1 | BAD67157 | Ohgushi et al | 2004 | Bt sotto
Cry30Ca2 | ACU24781 | Sun and Park | 2009 | Bt jegathesan 367
Cry30Da1 | EF095955 | Shu et al | 2006 | Bt Y41 | No NCBI link July09
Cry30Db1 | BAE80088 | Kishida et al | 2006 | Bt aizawai BUN1-14
Cry30Ea1 | ACC95445 | Fang et al | 2007 | Bt S2160-1
Cry30Ea2 | FJ499389 | Jun et al | 2008 | Bt Ywc2-8 | No NCBI link July09
Cry30Fa1 | ACI22625 | Tan et al | 2008 | Bt MC28
Cry30Ga1 | ACG60020 | Zhu et al | 2008 | Bt HS18-1
Cry31Aa1 | BAB11757 | Saitoh & Mizuki | 2000 | Bt 84-HS-1-11
Cry31Aa2 | AAL87458 | Jung and Cote | 2000 | Bt M15
Cry31Aa3 | BAE79808 | Uemori et al | 2006 | Bt B0195
Cry31Aa4 | BAF32571 | Yasutake et al | 2006 | Bt 79-25
Cry31Aa5 | BAF32572 | Yasutake et al | 2006 | Bt 92-10
Cry31Ab1 | BAE79809 | Uemori et al | 2006 | Bt B0195
Cry31Ab2 | BAF32570 | Yasutake et al | 2006 | Bt 31-5
Cry31Ac1 | BAF34368 | Yasutake et al | 2006 | Bt 87-29
Cry32Aa1 | AAG36711 | Balasubramanian et al | 2001 | Bt yunnanensis
Cry32Ba1 | BAB78601 | Takebe et al | 2001 | Bt
Cry32Ca1 | BAB78602 | Takebe et al | 2001 | Bt
Cry32Da1 | BAB78603 | Takebe et al | 2001 | Bt
Cry33Aa1 | AAL26871 | Kim et al | 2001 | Bt dakota
Cry34Aa1 | AAG50341 | Ellis et al | 2001 | Bt PS80JJ1 | Binary with Cry35Aa1
Cry34Aa2 | AAK64560 | Rupar et al | 2001 | Bt EG5899 | Binary with Cry35Aa2
Cry34Aa3 | AAT29032 | Schnepf et al | 2004 | Bt PS69Q | Binary with Cry35Aa3
Cry34Aa4 | AAT29030 | Schnepf et al | 2004 | Bt PS185GG | Binary with Cry35Aa4
Cry34Ab1 | AAG41671 | Moellenbeck et al | 2001 | Bt PS149B1 | Binary with Cry35Ab1
Cry34Ac1 | AAG50118 | Ellis et al | 2001 | Bt PS167H2 | Binary with Cry35Ac1
Cry34Ac2 | AAK64562 | Rupar et al | 2001 | Bt EG9444 | Binary with Cry35Ab2

Cry34Ac3 | AAT29029 | Schnepf et al | 2004 | Bt KR1369 | Binary with Cry35Ab3
Cry34Ba1 | AAK64565 | Rupar et al | 2001 | Bt EG4851 | Binary with Cry35Ba1
Cry34Ba2 | AAT29033 | Schnepf et al | 2004 | Bt PS201L3 | Binary with Cry35Ba2
Cry34Ba3 | AAT29031 | Schnepf et al | 2004 | Bt PS201HH2 | Binary with Cry35Ba3
Cry35Aa1 | AAG50342 | Ellis et al | 2001 | Bt PS80JJ1 | Binary with Cry34Aa1
Cry35Aa2 | AAK64561 | Rupar et al | 2001 | Bt EG5899 | Binary with Cry34Aa2
Cry35Aa3 | AAT29028 | Schnepf et al | 2004 | Bt PS69Q | Binary with Cry34Aa3
Cry35Aa4 | AAT29025 | Schnepf et al | 2004 | Bt PS185GG | Binary with Cry34Aa4
Cry35Ab1 | AAG41672 | Moellenbeck et al | 2001 | Bt PS149B1 | Binary with Cry34Ab1
Cry35Ab2 | AAK64563 | Rupar et al | 2001 | Bt EG9444 | Binary with Cry34Ac2
Cry35Ab3 | AY536891 | AAT29024 | 2004 | Bt KR1369 | Binary with Cry34Ab3
Cry35Ac1 | AAG50117 | Ellis et al | 2001 | Bt PS167H2 | Binary with Cry34Ac1
Cry35Ba1 | AAK64566 | Rupar et al | 2001 | Bt EG4851 | Binary with Cry34Ba1
Cry35Ba2 | AAT29027 | Schnepf et al | 2004 | Bt PS201L3 | Binary with Cry34Ba2
Cry35Ba3 | AAT29026 | Schnepf et al | 2004 | Bt PS201HH2 | Binary with Cry34Ba3
Cry36Aa1 | AAK64558 | Rupar et al | 2001 | Bt |
Cry37Aa1 | AAF76376 | Donovan et al | 2000 | Bt | Binary with Cry23Aa
Cry38Aa1 | AAK64559 | Rupar et al | 2000 | Bt |
Cry39Aa1 | BAB72016 | Ito et al | 2001 | Bt aizawai |
Cry40Aa1 | BAB72018 | Ito et al | 2001 | Bt aizawai |
Cry40Ba1 | BAC77648 | Ito et al | 2003 | Bun1-14 |
Cry40Ca1 | EU381045 | Shu et al | 2008 | Bt Y41 | No NCBI link July09
Cry40Da1 | ACF15199 | Zhang et al | 2008 | Bt S2096-2 |
Cry41Aa1 | BAD35157 | Yamashita et al | 2003 | Bt A1462 |
Cry41Ab1 | BAD35163 | Yamashita et al | 2003 | Bt A1462 |
Cry42Aa1 | BAD35166 | Yamashita et al | 2003 | Bt A1462 |
Cry43Aa1 | BAD15301 | Yokoyama and
Tanaka | 2003 | P. lentimorbus
semadara |
Cry43Aa2 | BAD95474 | Nozawa | 2004 | P. popilliae
popilliae |
Cry43Ba1 | BAD15303 | Yokoyama and
Tanaka | 2003 | P. lentimorbus
semadara |
Cry43-like | BAD15305 | Yokoyama and
Tanaka | 2003 | P. lentimorbus
semadara |
Cry44Aa | BAD08532 | Ito et al | 2004 | Bt entomocidus
INA288 |
Cry45Aa | BAD22577 | Okumura et al | 2004 | Bt 89-T-34-22 |
Cry46Aa | BAC79010 | Ito et al | 2004 | Bt dakota |
Cry46Aa2 | BAG68906 | Ishikawa et al | 2008 | Bt A1470 |
Cry46Ab | BAD35170 | Yamagiwa et al | 2004 | Bt |

Cry47Aa | AAY24695 | Kongsuwan et al | 2005 | Bt CAA890
Cry48Aa | CAJ18351 | Jones and Berry | 2005 | Bs IAB59 | binary with 49Aa
Cry48Aa2 | CAJ86545 | Jones and Berry | 2006 | Bs 47-6B | binary with 49Aa2
Cry48Aa3 | CAJ86546 | Jones and Berry | 2006 | Bs NHA15b | binary with 49Aa3
Cry48Ab | CAJ86548 | Jones and Berry | 2006 | Bs LP1G | binary with 49Ab1
Cry48Ab2 | CAJ86549 | Jones and Berry | 2006 | Bs 2173 | binary with 49Aa4
Cry49Aa | CAH56541 | Jones and Berry | 2005 | Bs IAB59 | binary with 48Aa
Cry49Aa2 | CAJ86541 | Jones and Berry | 2006 | Bs 47-6B | binary with 48Aa2
Cry49Aa3 | CAJ86543 | Jones and Berry | 2006 | BsNHA15b | binary with 48Aa3
Cry49Aa4 | CAJ86544 | Jones and Berry | 2006 | Bs 2173 | binary with 48Ab2
Cry49Ab1 | CAJ86542 | Jones and Berry | 2006 | Bs LP1G | binary with 48Ab1
Cry50Aa1 | BAE86999 | Ohgushi et al | 2006 | Bt sotto
Cry51Aa1 | ABI14444 | Meng et al | 2006 | Bt F14-1
Cry52Aa1 | EF613489 | Song et al | 2007 | Bt Y41 | No NCBI link July09
Cry52Ba1 | FJ361760 | Jun et al | 2008 | Bt BM59-2 | No NCBI link July09
Cry53Aa1 | EF633476 | Song et al | 2007 | Bt Y41 | No NCBI link July09
Cry53Ab1 | FJ361759 | Jun et al | 2008 | Bt MC28 | No NCBI link July09
Cry54Aa1 | ACA52194 | Tan et al | 2009 | Bt MC28
Cry55Aa1 | ABW88931 | Guo et al | 2008 | YBT 1518
Cry55Aa2 | AAE33526 | Bradfisch et al | 2000 | BT Y41
Cry56Aa1 | FJ597621 | Jun & Furong | 2008 | Bt Ywc2-8 | No NCBI link July09
Cry56Aa2 | GQ483512 | Guan Peng et al | 2009 | Bt G7-1 | No NCBI link Aug09
Cry57Aa1 | ANC87261 | Noguera & Ibarra | 2009 | Bt kim
Cry58Aa1 | ANC87260 | Noguera & Ibarra | 2009 | Bt entomocidus
Cry59Aa1 | ACR43758 | Noguera & Ibarra | 2009 | Bt kim LBIT-980

Vip3Aa1 | Vip3Aa | AAC37036 | Estruch et al | 1996 | PNAS 93, 5389-5394 | AB88 |
Vip3Aa2 | Vip3Ab | AAC37037 | Estruch et al | 1996 | PNAS 93, 5389-5394 | AB424 |
Vip3Aa3 | Vip3Ac | | Estruch et al | 2000 | US 6137033 Oct 2000 | |
Vip3Aa4 | PS36A Sup | AAR81079 | Feitelson et al | 1998 | US 6656908 Dec 2003 | Bt PS36A | WO9818932(A2,A3) 7 May 1998
Vip3Aa5 | PS81F Sup | AAR81080 | Feitelson et al | 1998 | US 6656908 Dec 2003 | Bt PS81F | WO9818932(A2,A3) 7 May

| | | | | | | 1998
Vip3Aa6 | Jav90 Sup | AAR81081 | Feitelson et al | 1998 | US 6656908 Dec 2003 | Bt | WO9818932(A2,A3) 7 May 1998
Vip3Aa7 | Vip83 | AAK95326 | Cai et al | 2001 | unpublished | Bt YBT-833 |
Vip3Aa8 | Vip3A | AAK97481 | Loguercio et al | 2001 | unpublished | Bt HD125 |
Vip3Aa9 | VipS | CAA76665 | Selvapandiyan et al | 2001 | unpublished | Bt A13 |
Vip3Aa10 | Vip3V | AAN60738 | Doss et al | 2002 | Protein Expr. Purif. 26, 82-88 | Bt |
Vip3Aa11 | Vip3A | AAR36859 | Liu et al | 2003 | unpublished | Bt C9 |
Vip3Aa12 | Vip3A-WB5 | AAM22456 | Wu and Guan | 2003 | unpublished | Bt |
Vip3Aa13 | Vip3A | AAL69542 | Chen et al | 2002 | Sheng Wu Gong Cheng Xue Bao 18, 687-692 | Bt S184 |
Vip3Aa14 | Vip | AAQ12340 | Polumetla et al | 2003 | unpublished | Bt tolworthi |
Vip3Aa15 | Vip3A | AAP51131 | Wu et al | 2004 | unpublished | Bt WB50 |
Vip3Aa16 | Vip3LB | AAW65132 | Mesrati et al | 2005 | FEMS Micro Lett 244, 353-358 | Bt |
Vip3Aa17 | Jav90 | | Feitelson et al | 1999 | US 6603063 Aug 2003 | Javelin 1990 | WO9957282(A2,A3) 11Nov 1999
Vip3Aa18 | | AAX49395 | Cai and Xiao | 2005 | unpublished | Bt 9816C |
Vip3Aa19 | Vip3ALD | DQ241674 | Liu et al | 2006 | unpublished | Bt AL |
Vip3Aa19 | Vip3A-1 | DQ539887 | Hart et al | 2006 | unpublished | |
Vip3Aa20 | Vip3A-2 | DQ539888 | Hart et al | 2006 | unpublished | |
Vip3Aa21 | Vip | ABD84410 | Panbangred | 2006 | unpublished | Bt aizawai |
Vip3Aa22 | Vip3A-LS1 | AAY41427 | Lu et al | 2005 | unpublished | Bt LS1 |
Vip3Aa23 | Vip3A-LS8 | AAY41428 | Lu et al | 2005 | unpublished | Bt LS8 |
Vip3Aa24 | | BI 880913 | Song et al | 2007 | unpublished | Bt WZ-7 |
Vip3Aa25 | | EF608501 | Hsieh et al | 2007 | unpublished | |
Vip3Aa26 | | EU294496 | Shen and Guo | 2007 | unpublished | Bt TF9 |
Vip3Aa27 | | EU332167 | Shen and Guo | 2007 | unpublished | Bt 16 |
Vip3Aa28 | | FJ494817 | Xiumei Yu | 2008 | unpublished | Bt JF23-8 |
Vip3Aa29 | | FJ626674 | Xieumei et al | 2009 | unpublished | Bt JF21-1 |
Vip3Aa30 | | FJ626675 | Xieumei et al | 2009 | unpublished | MD2-1 |
Vip3Aa31 | | FJ626676 | Xieumei et al | 2009 | unpublished | JF21-1 |

Vip3Aa32 |  | FJ626677 | Xieumei et al | 2009 | unpublished | MD2-1 |
. |  |  |  | . |  |  |
Vip3Ab1 | Vip3B | AAR40284 | Feitelson et al | 1999 | US 6603063
Aug 2003 | Bt KB59A4-6 | WO9957282(A
2,A3) 11Nov
1999
Vip3Ab2 | Vip3D | AAY88247 | Feng and Shen | 2006 | unpublished | Bt |
. |  |  |  | . |  |  |
Vip3Ac1 | PS49C |  | Narva et al | . | US
application
2004012871
6 |  |
. |  |  |  | . |  |  |
Vip3Ad1 | PS158C2 |  | Narva et al | . | US
application
2004012871
6 |  |
Vip3Ad2 | ISP3B | CAI43276 | Van Rie et al | 2005 | unpublished | Bt |
. |  |  |  | . |  |  |
Vip3Ae1 | ISP3C | CAI43277 | Van Rie et al | 2005 | unpublished | Bt |
. |  |  |  | . |  |  |
Vip3Af1 | ISP3A | CAI43275 | Van Rie et al | 2005 | unpublished | Bt |
Vip3Af2 | Vip3C | ADN08753 | Syngenta | . | WO
03/075655 |  |
. |  |  |  | . |  |  |
Vip3Ag1 | Vip3B | ADN08758 | Syngenta | . | WO
02/078437 |  |
Vip3Ag2 |  | FJ556803 | Audtho et al | 2008 |  | Bt |
. |  |  |  | . |  |  |
Vip3Ah1 | Vip3S | DQ832323 | Li and Shen | 2006 | unpublished | Bt |
. |  |  |  |  |  |  |
Vip3Ba1 |  | AAV70653 | Rang et al | 2004 | unpublished |  |
. |  |  |  |  |  |  |
Vip3Bb1 | Vip3Z | ADN08760 | Syngenta | . | WO
03/075655 |  |
Vip3Bb2 |  | EF439819 | Akhurst et al | 2007 |  |  |

The invention also pertains to the following:
1. A transgenic plant comprising DNA encoding a Cry1Fa insecticidal protein and DNA encoding a second insecticidal protein selected from the group consisting of a Cry2Aa insecticidal protein and a Cry1I insecticidal protein.
2. A transgenic plant comprising DNA encoding a Cry1Fa insecticidal protein and DNA encoding a Cry1E insecticidal protein.
3. The transgenic plant of item 1 , said plant further comprising DNA encoding a third insecticidal protein, said third protein being selected from the group consisting of Cry1Be, Cry1Ab, and DIG-3.
4. The transgenic plant of item 3 , wherein said third protein is selected from the group consisting of Cry1Be, said plant further comprising DNA encoding fourth insecticidal protein selected from the group consisting of Cry1Ca, Cry1Da, and Vip3Ab.
5. The transgenic plant of item 2, said plant further comprising DNA encoding a third insecticidal protein, said third protein being selected from the group consisting of Cry1Be, Cry1Ca, Cry1Da, and Vip3Ab.
6. The transgenic plant of item 5 , wherein said third protein is selected from the group consisting of Cry1Be, said plant further comprising DNA encoding fourth insecticidal protein selected from the group consisting of Cry1Ab, Cry1I, Cry2A, and DIG-3.
7. Seed of a plant according to any of items 1-6, wherein said seed comprises said DNA.
8. A field of plants comprising non-Bt refuge plants and a plurality of plants according to any of items 1-6, wherein said refuge plants comprise less than 40% of all crop plants in said field.
9. The field of plants of item 8 , wherein said refuge plants comprise less than 30% of all the crop plants in said field.
10. The field of plants of item 8 , wherein said refuge plants comprise less than 20% of all the crop plants in said field.
11. The field of plants of item 8 , wherein said refuge plants comprise less than 10% of all the crop plants in said field.
12. The field of plants of item 8 , wherein said refuge plants comprise less than 5% of all the crop plants in said field.
13. The field of plants of item 8 , wherein said refuge plants are in blocks or strips.
14. A mixture of seeds comprising refuge seeds from non-Bt refuge plants, and a plurality of seeds of item 7 , wherein said refuge seeds comprise less than 40% of all the seeds in the mixture.
15. The mixture of seeds of item 14 , wherein said refuge seeds comprise less than 30% of all the seeds in the mixture.
16. The mixture of seeds of item 14 , wherein said refuge seeds comprise less than 20% of all the seeds in the mixture.
17. The mixture of seeds of item 14 , wherein said refuge seeds comprise less than 10% of all the seeds in the mixture.
18. The mixture of seeds of item 14, wherein said refuge seeds comprise less than 5% of all the seeds in the mixture.
19. A method of managing development of resistance to a *Cry* protein by an insect, said method comprising planting seeds to produce a field of plants of item 8 .
20. A field of any of item 8-13 , wherein said plants occupy more than 10 acres.
21. A plant of any of item 1-6, wherein said plant is selected from the group consisting of corn, soybeans, and cotton.
22. The plant of item 21 , wherein said plant is a maize plant.
23. A method of controlling a European cornborer insect by contacting said insect with a Cry1F insecticidal protein and a second insecticidal protein selected from the group consisting of a Cry2Aa insecticidal protein and a Cry1I insecticidal protein.
24. An isolated polynucleotide that encodes a Cry1F insecticidal protein in combination with a second insecticidal protein selected from the group consisting of a Cry2Aa insecticidal protein and a Cry1I insecticidal protein.
25. A method of making a Cry1F insecticidal protein in combination with a second insecticidal protein selected from the group consisting of a Cry2Aa insecticidal protein and a Cry1I insecticidal protein.
26. A method of controlling a fall armyworm insect by contacting said insect with a Cry1F insecticidal protein and a Cry1E insecticidal protein.
27. An isolated polynucleotide that encodes a Cry1F insecticidal protein in combination with a Cry1E insecticidal protein.
28. A method of making a Cry1F insecticidal protein in combination with a Cry1E insecticidal protein.
29. A plant cell of a plant of item 1 , wherein said Cry1Fa insecticidal protein is at least 99% identical with SEQ ID NO:1, said Cry2Aa insecticidal protein is at least 99% identical with SEQ ID NO:2, and said Cry1I insecticidal protein is at least 99% identical with SEQ ID NO:3.
30. A plant cell of a plant of item 2, wherein said Cry1Fa insecticidal protein is at least 99% identical with SEQ ID NO:1, and said Cry1E insecticidal protein is at least 99% identical with SEQ ID NO:4.

## Claims

1. A transgenic plant comprising DNA encoding and expressing a Cry1Fa insecticidal protein and DNA encoding and expressing a Cry1Ea insecticidal protein.

2. The transgenic plant of claim 1, further comprising DNA encoding and expressing a third insecticidal protein selected from the group consisting of Cry1Be, Cry1Ca, Cry1Da, and Vip3Ab

3. The transgenic plant of claim 1, wherein the Cry1Fa insecticidal protein is at least 99% identical with SEQ ID NO:1, and wherein the Cry1Ea insecticidal protein is at least 99% identical with SEQ ID NO:4.

4. A seed of the transgenic plant of claim 1, wherein the seed comprises the DNA encoding and expressing the Cry1Fa insecticidal protein, and the DNA encoding and expressing the Cry1Ea insecticidal protein.

5. A mixture of seeds comprising a plurality of refuge seeds from non-*Bt* refuge plants, and a plurality of the seeds of the transgenic plant according to claim 4, wherein the seeds of the transgenic plant comprise the DNA encoding and expressing the Cry1Fa insecticidal protein and the DNA encoding and expressing the Cry1Ea insecticidal protein, and wherein the non-*Bt* refuge seeds comprise less than 40% of all seeds in the mixture.

6. A field of plants comprising a plurality of the transgenic plants according to any one of claims 1-3.

7. The field of plants of claim 6, further comprising a plurality of non-*Bt* refuge plants.

8. The field of plants of claim 7, wherein the non-*Bt* refuge plants comprise less than 40% of all crop plants in the field.

9. The field of plants of claim 7, wherein the non-*Bt* refuge plants comprise less than 30% of all crop plants in the field.

10. The field of plants of claim 7, wherein the non-*Bt* refuge plants comprise less than 20% of all crop plants in the field.

11. The field of plants of claim 7, wherein the non-*Bt* refuge plants comprise less than 10% of all crop plants in the field.

12. The field of plants of claim 7, wherein the non-*Bt* refuge plants comprise less than 5% of all crop plants in the field.

13. The field of plants of claim 7, wherein the non-*Bt* refuge plants are in blocks or strips.

14. A method of controlling a fall armyworm insect by contacting the insect with a CrylFa insecticidal protein and a CrylEa insecticidal protein.

15. An isolated polynucleotide of the transgenic plant of claim 1 that encodes the CrylFa insecticidal protein in combination with the CrylEa insecticidal protein.

16. A plant cell of the transgenic plant of claim 1.
